(19) **Europäisches Patentamt European Patent Office Office européen des brevets**

(11) **EP 4 710 956 A1**

(12) **EUROPEAN PATENT APPLICATION**
published in accordance with Art. 153(4) EPC

(43) Date of publication:
**18.03.2026 Bulletin 2026/12**

(21) Application number: **24803520.6**

(22) Date of filing: **09.05.2024**

(51) International Patent Classification (IPC):
**A61L 15/28** (2006.01)     **A61L 15/26** (2006.01)
**A61L 15/64** (2006.01)

(52) Cooperative Patent Classification (CPC):
**A61L 15/26; A61L 15/28; A61L 15/64**

(86) International application number:
**PCT/JP2024/017249**

(87) International publication number:
**WO 2024/232411 (14.11.2024 Gazette 2024/46)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **09.05.2023   JP 2023077267**

(71) Applicant: **Kureha Corporation**
**Chuo-ku**
**Tokyo 103-8552 (JP)**

(72) Inventors:
- **SHIMADA, Hironao**
  **Tokyo 103-8552 (JP)**
- **SEKINE, Fujio**
  **Tokyo 103-8552 (JP)**
- **YAMASHITA, Yusuke**
  **Tokyo 103-8552 (JP)**
- **OHIRA, Yurika**
  **Tokyo 103-8552 (JP)**
- **TAKANOHASHI, Masato**
  **Tokyo 103-8552 (JP)**

(74) Representative: **Hoefer & Partner Patentanwälte
mbB**
**Pilgersheimer Straße 20**
**81543 München (DE)**

(54) **MEDICAL FILM**

(57)     Provided is a medical film that can enhance re-attachability even without forming a layer for controlling solubility. The medical film of the present invention has a ratio q1 (V1/V0) of a volume V1 after 10 seconds of immersion in a phosphate buffer (PB) having a pH of 6.0 and a concentration of 0.2 M to a volume V0 before the immersion of 1.5 or less.

# EP 4 710 956 A1

**Description**

## TECHNICAL FIELD

The present invention relates to a medical film.

## BACKGROUND ART

There has been known a medical film (adhesion barrier materials) that is attached to an organ after a surgical operation to prevent adhesion between the organ and another organ at the time of healing of a wound. A bioabsorbable material such as a crosslinked product of hyaluronic acid and carboxymethyl cellulose is used for such an adhesion barrier.

A medical film that is attached to an organ, such as an adhesion barrier, is required to have re-attachability by adjusting an attachment position after the medical film is attached to an organ surface. As such a medical film having improved re-attachability, Patent Document 1 describes an adhesion barrier including a base layer containing a water-soluble polymer, and a support layer formed on the surface of the base layer and containing an aliphatic ester. Patent Document 1 describes that the adhesion barrier is easily handled with tweezers or the like even in a wet state and is easily re-attached because its surface is provided with the support layer made of a water-insoluble aliphatic ester.

Patent Document 2 describes an adhesion barrier including a layer containing an aliphatic polyester and a plurality of layers formed on the surface thereof and containing a water-soluble polymer. Patent Document 2 describes that the adhesion barrier can control the solubility of the surface in a wet state after attachment because of the water-soluble polymer layers, and is likely to maintain the shape even after attachment to an adherend.

### Citation List

### Patent Document

Patent Document 1: WO 2011/081162
Patent Document 2: WO 2017/164264

## SUMMARY OF INVENTION

### TECHNICAL PROBLEM

Medical films having an adjusted solubility immediately after attachment to an organ have been known similarly to those described in Patent Document 1 and Patent Document 2. Meanwhile, a medical film including a layer for controlling solubility provided on a surface, such as those described in these documents, tends to have a lower absorbability into a living body due to a large thickness or tends to be expensive.

The present invention has been completed in light of the problems described above, and an object of the present invention is to provide a medical film that can enhance re-attachability even without forming a layer for controlling solubility.

### SOLUTION TO PROBLEM

One embodiment of the present invention for solving the problems described above relates to a medical film described below in [1] to [7].

[1] A medical film containing a crosslinked polymer material and a biodegradable resin, wherein
the medical film has a ratio $q_1$ ($V_1/V_0$) of a volume $V_1$ after 10 seconds of immersion in a phosphate buffer (PB) having a pH of 6.0 and a concentration of 0.2 M to a volume $V_0$ before the immersion of 1.5 or less.
[2] The medical film according to [1], wherein the medical film has a ratio $q_2$ ($V_2/V_0$) of a volume $V_2$ after 60 seconds of immersion in a phosphate buffer (PB) having a pH of 6.0 and a concentration of 0.2 M to a volume $V_0$ before the immersion of 1.9 or less.
[3] The medical film according to [1] or [2], wherein the medical film has a ratio $q_3$ ($V_3/V_0$) of a volume $V_3$ after 900 seconds of immersion in a phosphate buffer (PB) having a pH of 6.0 and a concentration of 0.2 M to a volume $V_0$ before the immersion of 1.8 or more.
[4] The medical film according to any of [1] to [3], wherein the medical film contains 5 parts by mass or more and 95 parts by mass or less of the biodegradable resin relative to 100 parts by mass of the crosslinked polymer material.

[5] The medical film according to any of [1] to [4], wherein the biodegradable resin is a polymer in which a proportion of a polymer including a constituent unit of glycolic acid is 10 mass% or more and 100 mass% or less relative to a mass of the entire biodegradable resin.

[6] The medical film according to any of [1] to [5], wherein the medical film has a thickness of 15 $\mu$m or more and 500 $\mu$m or less.

[7] The medical film according to any of [1] to [6], wherein the medical film is an adhesion barrier.

## ADVANTAGEOUS EFFECTS OF INVENTION

According to the present invention, there is provided a medical film that can enhance re-attachability even without forming a layer for controlling solubility.

## DESCRIPTION OF EMBODIMENTS

Medical Film

An embodiment of the present invention relates to a medical film containing a crosslinked polymer material and a biodegradable resin (hereinafter may be referred to simply as "medical film").

Crosslinked Polymer Material

The crosslinked polymer material is a polymer material that swells when immersed in ultrapure water at 15 to 25°C. The crosslinked polymer material is a polymer material obtained by physically crosslinking or chemically crosslinking the same type of or different types of polymer materials and is typically a polymer material in a gel form. The crosslinked polymer material is preferably made of a polymer material having solubility after swelling in water.

The crosslinked polymer material can be, for example, a crosslinked body of an anionic polymer material. The anionic polymer material may be a polymer material having a repeating structure containing an anionic group, or may be a polymer material obtained by chemically modifying a polymer compound with an anionic substituent. The anionic polymer material may be a polymer material containing a cationic substituent besides the anionic substituent. The anionic group may be a carboxyl group, a hydroxy group, a sulfo group, and the like. The anionic group may partially form a salt with an alkali metal such as sodium or potassium or an alkaline earth metal such as calcium or magnesium.

The polymer material may be a polysaccharide, a protein, or a synthetic polymer. Among these, a polysaccharide and a protein are preferred.

Examples of the saccharide as the anionic polymer material may include natural saccharides, such as pullulan, alginic acid, hyaluronic acid, chondroitin acid, dextran acid, and pectin, and saccharides containing an anionic group introduced by modification, such as carboxymethyl amylose, carboxymethyl cellulose, carboxymethyl dextran, carboxymethyl starch, cellulose sulfate, and dextran sulfate. Among these, alginic acid, hyaluronic acid, carboxymethyl amylose, and carboxymethyl cellulose are preferred, from the viewpoint of easy handling.

Examples of the protein as the anionic polymer material include gelatin, collagen, albumin, fibrin, and chemically modified products of these. The protein may be a polymer of an amino acid containing an anionic group (polyamino acid), such as polyglutamic acid or polyaspartic acid. Among these, acid-treated gelatin containing a carboxyl group formed by hydrolysis of an acid amide is preferred.

The anionic polymer material can be identified by any instrumental analysis, acid titration, or a method using these in combination. For example, the identification can be performed by a method of using a spectroscope, such as FT-IR; a method of analyzing a solution obtained by heating to such a degree that a polymer material is dissolved by NMR using a deuterated dimethyl sulfoxide (deuterated DMSO) or the like; or a method of appropriately using an analytical instrument such as liquid chromatography for a solution obtained by heating to such a degree that a polymer material is dissolved using DMSO or the like.

The crosslinking of the polymer material may be physical crosslinking formed by introduction of a cationic substituent into a polyanionic polymer material, or may be chemical crosslinking formed by allowing polymer materials to be directly reacted or reacted via another molecule. The crosslinking of the polymer material can be performed by a method of subjecting the polymer material described above to a crosslinking treatment, such as an ultraviolet treatment, a heat treatment, or a crosslinking agent treatment. Among these, a crosslinking agent treatment is preferred.

Examples of the crosslinking agent used in the crosslinking agent treatment include ethyl-3-(3-dimethylaminopropyl)carbodiimide (EDC), N,N'-dicyclohexylcarbodiimide, N,N'-diisopropylcarbodiimide, and N-cyclohexyl-N'-(2-morpholinoethyl)carbodiimide metho-p-toluenesulfonate. A cationic group can be introduced into the polyanionic polymer material by performing a treatment with such a crosslinking agent. Molecules of the polymer material can be physically crosslinked by intermolecular interaction between an anionic group of a molecule and a cationic group of another molecule. Among these, EDC is preferred from the viewpoint of easy handling.

The crosslinking treatment by a crosslinking agent can be performed by dissolving the polymer material in water or an aqueous medium, adding a crosslinking agent to the solution, and stirring the mixture. In this case, the conditions of the water or aqueous solution may be adjusted based on the type of the crosslinking agent. For example, when EDC is used as the crosslinking agent, preferably, the pH is made low (e.g., pH of 4.0 to 5.5) during reaction by appropriately adding an acid such as 0.01 M hydrochloric acid to dissociate an anionic group in a polyanionic polymer.

The amount of the crosslinking agent is preferably 0.5 eq. or more and 10 eq. or less, more preferably 1 eq. or more and 6 eq. or less, and even more preferably 2 eq. or more and 5 eq. or less, relative to the amount of reactive functional groups of the polymer material (e.g., hyaluronic acid). As described below, when the amount of the crosslinking agent is increased, the initial swelling degree of the medical film can be reduced.

The crosslinking may be formed between a plurality of different types of polymer materials. In this case, the crosslinking may be chemical crosslinking that can form chemical bonding by chemically reacting these polymer materials, or may be physical crosslinking by enhancing the intermolecular interaction with a crosslinking agent or the like. For example, from the viewpoint of safety, a material obtained by crosslinking a combination of hyaluronic acid and carboxymethyl cellulose can be used as the crosslinked polymer material.

Biodegradable Resin

The biodegradable resin is a resin that is degraded in a living body. The biodegradable resin can be a biodegradable polyester. Examples of the biodegradable polyester include biodegradable aliphatic polyesters, and specific examples include polyglycolic acid, polylactic acid, polycaprolactone, polydioxanone, and copolymers of these. Among these, a (co)polymer of glycolic acid, such as a homopolymer of glycolic acid or a copolymer including a structure derived from glycolic acid, is preferred because of a high rate of decomposition in a living body.

The (co)polymer of glycolic acid may be a homopolymer of glycolic acid or may be a copolymer of glycolic acid and another monomer. The copolymer can be a copolymer of glycolic acid and a hydroxycarboxylic acid, such as lactic acid, hydroxypropionic acid, hydroxybutyric acid, hydroxyvaleric acid, hydroxycaproic acid, or hydroxybenzoic acid, or a lactone such as caprolactone, or a water-soluble polymer having safety to a living body, such as polyethylene glycol. From the viewpoint of lowering the initial degree of swelling, the proportion of the constituent unit derived from glycolic acid in the biodegradable resin is preferably 5 mass% or more and 100 mass% or less, more preferably 20 mass% or more and 99 mass% or less, and even more preferably 40 mass% or more and 95 mass% or less.

The type of the biodegradable resin can be identified by using, for example, NMR. In a case where the biodegradable resin is a resin that does not dissolve in a solvent at norm temperature, such as a glycolic acid polymer, a sample for measurement can be prepared by heating and dissolving in dehydrated deuterated DMSO. Another analytical instrument such as FT-IR or GC-MS may be used in combination.

Such a biodegradable resin slowly releases an acid by hydrolysis when the medical film is attached to an organ and brought into contact with water. The slowly released acid lowers the degree of swelling of the medical film in an early stage after attachment to reduce adhesion to the organ, whereby the re-attachability of the medical film can be enhanced. It is conceived that, because the decomposition of the crosslinked polymer material is delayed by the slowly released acid, the strength of the medical film is maintained, and the re-attachability of the medical film can be enhanced.

The number average molecular weight of the biodegradable resin is preferably 1000 or more and 150000 or less, more preferably 5000 or more and 100000 or less, and even more preferably 10000 or more and 50000 or less. The weight average molecular weight of the biodegradable resin is preferably 2000 or more and 300000 or less, more preferably 10000 or more and 200000 or less, and even more preferably 20000 or more and 100000 or less. When the number average molecular weight and the weight average molecular weight are lower, the slow-release performance of the acid can be enhanced, and the adhesion and film strength of the medical film can be maintained after the elapse of time following the attachment. In a case where the biodegradable resin is in a particulate form, when the number average molecular weight and the weight average molecular weight are lower, the biodegradable resin is readily pulverized, and particles having a smaller particle size can be obtained. As described below, a decrease in particle size enhances the effect of reducing the adhesion of the medical film to an organ in an early stage after attachment.

The number average molecular weight and the mass average molecular weight of the biodegradable resin refers to a value obtained by separating the biodegradable resin from the medical film. The number average molecular weight is a value measured in accordance with ISO 16014-1:2012. Specifically, the measurement is performed by gel permeation chromatography (GPC) (Shodex GPC-104, available from Resonac Corporation; detector: RI; columns: HFIP-606M $\times$ 2) using, as a solvent, a solution prepared by dissolving $CF_3COONa$ in hexafluoropropanol (HFIP) at a concentration of 5 mM and using poly(methyl methacrylate) (PMMA) as a reference substance.

The biodegradable resin may be in a particulate form or a fibrous form. When the biodegradable resin is in a fibrous form, the biodegradable resin may be in the form of monofilament, multifilament, nonwoven fabric, woven fabric, knitted fabric, braided fabric, or the like.

When the biodegradable resin is in a particulate form, the average particle size of the biodegradable resin is not particularly limited, but is preferably 0.1 $\mu$m or more and 30 $\mu$m or less, more preferably 1 $\mu$m or more and 20 $\mu$m or less, and even more preferably 1 $\mu$m or more and 15 $\mu$m or less. A larger average particle size facilitates handling of the biodegradable resin during production of the film. When the average particle size is larger, the surface area of a particle is larger, and the area of contact with the crosslinked polymer material is larger. Thus, the slow-release performance of the acid is increased, resulting in enhancement of the effect of reducing the adhesion of the medical film to an organ in an early stage after attachment by the biodegradable resin. The average particle size of the biodegradable resin refers to a value in the medical film. A digital image is taken by observation at a magnification of 100 times using a digital microscope (available from Keyence Corporation). The taken image of the measurement region is read into image analysis software MIPAR (available from Light Stone), and the particle size is determined as an equivalent circle diameter calculated based on the area of a particle image of the biodegradable resin. "Smart Cluster" is set to "Fill Type: Class", "Classes: 4 to 10", "Edge Type: Dark to Bright", "Edge Clean: 0 to 5", and "Speed: 2". "Basic Threshold" is set to "Value: 80 to 150". "Reject Features" is set to "Measurement: Area", "Target: Objects", "Edges: Include", "Units: $\mu$m$^2$", "Threshold Value: 100", and "Type: Reject Features</=".

When the biodegradable resin is in a nonwoven fabric form, the basis weight of the biodegradable resin is preferably 1.2 g/m$^2$ or more and 100.0 g/m$^2$ or less, more preferably 3.4 g/m$^2$ or more and 65.0 g/m$^2$ or less, and even more preferably 5.0 g/m$^2$ or more and 31.5 g/m$^2$ or less. A larger basis weight facilitates handling and can further reduce the initial swellability.

The content of the biodegradable resin is preferably 5 parts by weight or more and 95 parts by weight or less, more preferably 15 parts by weight and 85 parts by weight or less, and even more preferably 20 parts by weight or more and 70 parts by weight or less, when the content of the crosslinked polymer material is 100 parts by weight.

The content of the biodegradable resin contained in the medical film can be calculated based on the amount of the biodegradable resin added during medical film production or can be calculated by extracting the biodegradable resin from the medical film and measuring the mass of the biodegradable resin. The measurement of the mass of the biodegradable resin by extraction from the medical film can be performed by, for example, the following method. The medical film is treated by an acid-containing solution at normal temperature to decompose the crosslinked polymer material and dissolving the crosslinked polymer material in the solution. The obtained acid-containing solution is washed with water at low temperature while the solution is suction-filtered to remove the crosslinked polymer material, and the solid content is further washed with acetone. The obtained solid content is dried in a vacuum dryer set at 40°C for 5 minutes, and the weight of the obtained solid content is measured.

Additional Component

The medical film may contain a lubricant, an emulsifier, a coloring agent, an antioxidant, an anti-weathering agent, a thermal stabilizer, a crystal nucleating agent, a UV absorber, a coloring agent, and an antimicrobial agent. The content of such a component is preferably 0 mass% or more and 20 mass% or less, more preferably 0 mass% or more and 10 mass% or less, and even more preferably 0 mass% or more and 5 mass% or less, relative to the total mass of the medical film.

Swellability of Medical Film

The medical film has low initial swellability when brought into contact with water. Specifically, the medical film has a ratio q1 (V1/V0) (degree of swelling) of a volume V1 after 10 seconds of immersion in a phosphate buffer (PB) having a pH of 6.0 and a concentration of 0.2 M to a volume V0 before the immersion of 1.5 or less. Furthermore, the medical film preferably has a ratio q2 (V2/V0) of a volume V2 after 60 seconds of immersion in the phosphate buffer (PB) described above to the volume V0 before the immersion of 1.9 or less. In this case, the amount of the phosphate buffer needs to be an amount that enables complete immersion of the medical film. For example, a 10 mL vial is used, and 1 cm$^3$ of the medical film is completely immersed in 5 mL of the phosphate buffer, to evaluate the swellability.

For example, Seprafilm, which is a commercially available adhesion barrier (medical film), has a ratio q1 (V1/V0) of a volume V1 after 10 seconds of immersion in the phosphate buffer (PB) described above to the volume V0 before the immersion of approximately 1.7. The medical film that has high initial water absorbability and readily swells as described above exhibits high adhesion to an organ in an early stage after attachment, and the strength is likely to decrease immediately after the attachment. Thus, the medical film attached to an organ is difficult to be released, and when an attempt is made to forcibly peel off the medical film, re-attachment may often become impossible due to deformation.

In contrast, when the initial water absorbability of the medical film is made low to make the medical film difficult to be swollen, the adhesion to an organ in an early stage after attachment can be reduced, and release from the organ can be facilitated. In addition, deformation is less likely to occur when the medical film is released from the organ. Furthermore, even when the medical film is curled after release from the organ, the curled film can be easily flattened. Thus, such a medical film is easily re-attached to the organ.

For example, when the medical film is used in laparotomy, it is assumed that the medical film is attached to a place different from the target organ surface, released after several tens of seconds, and then re-attached. Thus, the medical film having a low degree of swelling after 10 seconds or 30 seconds of immersion can be suitably used particularly for laparotomy. From the viewpoints described above, the medical film has a ratio q1 (V1/VO) of a volume V1 after 10 seconds of immersion in the phosphate buffer (PB) described above to the volume V0 before the immersion of preferably 1.5 or less, more preferably 1.3 or less, and even more preferably 1.2 or less. Furthermore, the medical film has a ratio q4 (V4/V0) of a volume V4 after 30 seconds of immersion in the phosphate buffer (PB) described above to the volume V0 before the immersion of preferably 1.6 or less, more preferably 1.5 or less, and even more preferably 1.3 or less. The lower limit of each of the volume ratios q1 and q4 is not particularly limited and can be 1.0 or more.

When the medical film is used for laparoscopic surgery, it is assumed that visual field may be limited compared to laparotomy, the film may be operated with a forceps or the like, and thus more time may be required for re-attachment compared to the case of laparotomy. Thus, the medical film having a low degree of swelling after 60 seconds of immersion can be suitably used particularly for laparoscopic surgery. From the viewpoints described above, the medical film has a ratio q2 (V2/V0) of a volume V2 after 60 seconds of immersion in the phosphate buffer (PB) described above to the volume V0 before the immersion of preferably 1.9 or less, more preferably 1.7 or less, and even more preferably 1.5 or less. Furthermore, the medical film has a ratio q5 (V5/V0) of a volume V5 after 90 seconds of immersion in the phosphate buffer (PB) described above to the volume V0 before the immersion of preferably 1.6 or less, more preferably 1.5 or less, and even more preferably 1.3 or less. The lower limit of each of the volume ratios q2 and q5 is not particularly limited and can be 1.0 or more. Furthermore, a film may curl at the time of release of once attached film and re-attachment of the film. Even in the case of spreading of a film, the film is in an environment where swelling proceeds due to contact of the film with water. However, when the volume ratio q2 or q5 is in the preferred range described above, the film is readily spread.

Meanwhile, from the viewpoint of suppressing the position shift of the medical film due to deformation of the organ or the like after attachment to a right position, the medical film preferably adequately swells after the elapse of a predetermined time following the attachment. From the viewpoints described above, the medical film has a ratio q6 (V6/V0) of a volume V6 after 300 seconds of immersion in the phosphate buffer (PB) described above to the volume V0 before the immersion of preferably 1.6 or more, and more preferably 1.8 or more. Furthermore, the medical film has a ratio q3 (V3/V0) of a volume V3 after 900 seconds of immersion in the phosphate buffer (PB) described above to the volume V0 before the immersion of preferably 1.8 or more, more preferably 1.9 or more, and even more preferably 2.0 or more. The upper limit of the degree of swelling during such a period is not particularly limited, but can be 6.5 or less.

The medical film has the same degree of swelling after 900 seconds of immersion even when pH is changed, and no problems occur in attachment to a living body.

The volumes V1 to V6 described above can be determined by the following method.

Aside of a film is taken as the x-axis, another side that is in a perpendicular direction to the x-axis of the film is taken as the y-axis, and a vertical direction with respect to a plane including the x-axis and the y-axis is taken as the z-axis. In a case where the thickness of the medical film is thin, the thickness after immersion is difficult to measure. Thus, the volume of the medical film in the present description is calculated assuming that increase rates are all the same for the directions of the x-axis, the y-axis, and the z-axis. Specifically, for measurement of the volume V0 before the immersion, the medical film and a ruler are arranged together on a blue board in such a manner that the length of a side of the medical film is made clear, and they are photographed in such a manner that the contour of the sample is clear. The lengths of the four sides on the identical plane of the medical film measured using the obtained image are defined as a1, a2, a3, and a4, the average value $l_0$ of the lengths of the four sides is determined, and the volume V0 before the immersion is determined by Equation 1.

[Math. 1]

## Equation 1

$$V0 = \left(\frac{a1 + a2 + a3 + a4}{4}\right)^3 = \left(l_0\right)^3$$

Similarly, for each of the volumes (V1 to V6) after the immersion in the phosphate buffer, the medical film and a ruler are arranged together on a blue board in such a manner that the length of a side of the medical film is made clear, and they are photographed in such a manner that the contour of the sample is clear. The lengths of the four sides of the plane identical to the plane used for V0 measurement, which are determined using the obtained image, are defined as b1, b2, b3, and b4, the average value $l_1$ of the lengths of the four sides is determined, and each of the volumes after the immersion is determined by Equation 2. Equation 2 shows the method of determining the volume V1 after 10 seconds of the immersion, and the volumes V2 to V6 can be determined similarly.

[Math. 2]

## Equation 2

$$V1 = \left(\frac{b1 + b2 + b3 + b4}{4}\right)^3 = \left(1_1\right)^3$$

The volume ratios q1 to q6 determined based on these volumes V0 to V6 are each an average value obtained by three measurements using the medical film produced by the same formulation. Even when the plan view of the medical film is not substantially rectangular, the volume ratios q1 to q6 are determined based on the similar idea, i.e., on the assumption that the increase rate in each of the directions in the plan view is the same as the increase rate in the thickness direction.

Other Properties of Medical Film

The medical film has a high initial strength immediately after being wet. Specifically, when a sample cut into a strip form having a long side of 40 mm and a short side of 10 mm is fixed to chucks in such a manner that the distance between chucks becomes 20 mm, a 10 mm $\times$ 10 mm center portion of one surface of the sample is brought into contact with ultrapure water for 10 seconds, and then the sample is pulled to both sides at 0.1 mm/sec, the maximum tensile strength can be 1.50 MPa or more, and is preferably 2.00 MPa or more, and more preferably 6.00 MPa or more. The upper limit of the maximum tensile strength described above is not particularly limited, but can be 300.00 MPa or less.

The medical film has a high adhesive strength to an organ. Specifically, the medical film is cut into a strip form having a long side of 50 mm and a short side of 10 mm, and a mending tape (product number: 810-1-18, available from 3M Japan Limited; base material: acetate film; adhesive agent: acrylic) is attached on a surface of the cut film opposite to a surface to be brought into contact with a simulated organ (silicone rubber sheet (thickness: 3 mm), product number: 6-611-05, available from As One Corporation), to prepare a sample. Using a creepmeter (RE2-33005B, available from Yamaden Co., Ltd.), 5 mm of a short side of the sample is fixed to a pulling chuck, and the simulated organ is fixed to the other pulling chuck. The simulated organ is wet with ultrapure water and is adhered closely to an area with a long side of 40 mm and a short side of 10 mm of the sample and allowed to stand for 900 seconds. Thereafter, the sample is pulled at 0.05 mm/sec. In this case, the adhesive strength can be more than 0.05 N, and is more preferably more than 1.60 N. When the medical film has such an adhesive strength, the position shift of the medical film due to deformation of an organ or the like after attachment to a right position can be suppressed.

The swellability of the medical film can be adjusted by controlling the thickness of the medical film, the crosslinking condition of the crosslinked polymer material, the dispersibility of the biodegradable resin (in a case of a particulate form), and the like.

For example, a larger thickness of the medical film makes the initial degree of swelling lower. The thickness of the medical film is preferably 15 $\mu$m or more and 500 $\mu$m or less, and more preferably 40 $\mu$m or more and 200 $\mu$m or less. From the viewpoint of providing both low initial degree of swelling and usability, the thickness of a composite layer is more preferably 50 $\mu$m or more and 150 $\mu$m or less.

The crosslinking condition of the crosslinked polymer material and the dispersibility of the biodegradable resin can be changed by appropriately adjusting production conditions of the medical film.

Applications

The medical film can be attached to an organ or the like, and starts to change the form (disintegrate) due to decomposition after the elapse of a predetermined time and is eventually absorbed in a living body. The period from the attachment to the start of form change can be adjusted to from 3 days to 14 days. The period from the attachment to the time at which visual recognition of the medical film becomes impossible due to absorption in the living body can be adjusted to from 14 days to 28 days. These periods can be adjusted based on the type of the crosslinked polymer material and the type of the biodegradable resin.

Because of the properties described above, the medical film can be used as a covering material for an affected part, specifically, can be used to prevent adhesion by covering a wound region caused by a surgical operation (adhesion barrier) or to prevent occurrence of an ulcer and occurrence of perforation. The medical film can be also used as a sustained-release base material of a medicine and a base material for tissue regeneration. Alternatively, the medical film can be used as a space holder for holding a space by application to a wall of a lumen in a living body.

Method for Producing Medical Film

The medical film can be produced as follows: a polymer material is crosslinked, and the crosslinked polymer material is combined with a biodegradable resin, to form a film.

The crosslinking of the polymer material may be performed by the method depending on the type of the polymer

EP 4 710 956 A1

material, for example, performed by an ultraviolet treatment, a heat treatment, or a crosslinking agent treatment. For example, the crosslinking with a crosslinking agent using EDC can be performed by adding EDC to a solution of a polymer material in water or an aqueous medium while the solution is stirred. In this case, the solution is preferably in an acidic condition (e.g., pH 4.0 to 5.5) by addition of an acid such as HCl. After the crosslinking treatment with the crosslinking agent, undesired substances are preferably removed by dialysis or the like.

Combining with the biodegradable resin may be performed by a method depending on the form and type of the biodegradable resin. For example, when the biodegradable resin is in a particulate form, a monofilament form, or a multifilament form, the biodegradable resin in such a form may be added to and stirred in a solution or dispersion containing the crosslinked polymer material. When the biodegradable resin is in the form of nonwoven fabric, woven fabric, knitted fabric, or braided fabric, the biodegradable resin and the crosslinked polymer material can be combined in a frame to which the solution or dispersion is casted during film formation.

The film formation may be performed by drying a composite body of the crosslinked polymer material and the biodegradable resin in the frame. The drying may be performed by air-drying at normal temperature or freeze-drying, or by removing the solvent by heating. Also, hot pressing (heating and pressurization) may be performed. The drying may be performed by a combination of a plurality of methods.

In this case, the initial degree of swelling of the medical film can be adjusted by controlling the crosslinking condition of the crosslinked polymer material and the dispersibility of the biodegradable resin by controlling the production conditions.

For example, when the stirring speed at the time of crosslinking the polymer material is made faster, the degree of crosslinking is made higher by promoting the reaction by increasing contact frequency of the molecules to be reacted, and the initial degree of swelling of the medical film can be made lower. Meanwhile, the stirring speed is preferably an appropriate speed from the viewpoint of preventing the cutting rate of the molecules from being faster than the formation rate of crosslinking by the reaction caused by intense shearing force applied to a crosslinked polymer gel due to the stirring, and from the viewpoint of preventing increase of the initial degree of swelling of the composite layer due to a lowered degree of crosslinking caused by a decrease in reaction efficiency due to generation of bubbles. From the viewpoint of achieving good balance between these, the stirring speed is preferably 50 rpm or more and less than 750 rpm, and more preferably 150 rpm or more and less than 700 rpm.

Furthermore, when the temperature of the solution is set lower at the time of the crosslinking reaction of the polymer, the initial degree of swelling of the medical film can be lowered. Crosslinking points can be appropriately formed by performing the reaction at a low temperature. This probably results in production of a medical film having a low initial degree of swelling due to an appropriately high degree of crosslinking and, meanwhile, having a degree of swelling that becomes higher as time passes due to the degree of crosslinking not being excessively high. Meanwhile, when the temperature of the solution is set excessively low, time is required for the reaction or the reaction does not proceed. Thus, from the viewpoints of allowing the reaction to proceed adequately within a short time period and forming appropriate crosslinking points, the crosslinking reaction temperature of the polymer is preferably 0°C or higher and lower than 60°C, and more preferably 0°C or higher and lower than 50°C.

By appropriately changing these production conditions, the initial degree of swelling of the composite layer can be adjusted to fall within the range described above.

Other embodiments

The above-described embodiment is an exemplary embodiment of the present invention, and it is needless to say that the present invention can include embodiments other than the above-described embodiment within the scope of the core technical idea thereof.

EXAMPLES

The present invention will be described in detail below based on Examples, but the present invention is not limited to these Examples.

As described hereinafter, the weight average molecular weight of a material described with the name of manufacturer is a value published by the manufacturer, and the weight average molecular weight of a material that does not specifically described is a value determined by gel permeation chromatography (GPC) using, as a solvent, a 5 mM $CF_3COONa$ in hexafluoropropanol (HFIP) solvent and calculated by using poly(methyl methacrylate) (PMMA) as a reference substance. Although the same results are obtained regardless of the instrument used, in the present Examples, Shodex GPC-104 (detector: RI; columns: HFIP-606M $\times$ 2) available from Resonac Corporation was used as the instrument.

8

Experiment 1

In Experiment 1, the relationships among the initial degree of swelling of a medical film, ease in re-attachment, ease in spreading after releasing, tensile strength, and adhesive strength were determined.

1. Production of Medical Film

1-1. Production of Crosslinked Polymer Material

1-1-1. Production of HA/CMC Condensation Product-1

1650 mg of hyaluronic acid (HA) (available from Kewpie Corporation; molecular weight: 2200000 to 2500000) and 750 mg of carboxymethyl cellulose (CMC) (available from Tokyo Chemical Industry Co., Ltd.; molecular weight: 250000; degree of etherification: 0.5 to 0.8) were weighed. HA was added to a beaker containing 300 mL of ultrapure water and dissolved therein, and then CMC was added and dissolved therein, to prepare a mixed solution. Thereafter, 0.1 N of HCl was added, and the pH of the content was adjusted to 4.50 to 5.20, to prepare an HA/CMC solution. 3180 mg of ethyl-3-(3-dimethylaminopropyl)carbodiimide (EDC) (available from Dojindo Laboratories) was weighed and dissolved in 7.5 mL of ultrapure water, to prepare an EDC solution. While the HA/CMC solution was stirred at a stirring speed of 600 rpm at 25°C, an appropriate amount of 0.1 N HCl was added to the solution to adjust the pH to 4.70 to 5.10, and the entire amount of the EDC solution was added. After the addition, the resultant mixture was stirred for approximately 120 minutes until the pH remained unchanged, to produce a reaction solution. Finally, the reaction solution after the stirring was added to a dialysis membrane having a molecular weight cut off of 12000 to 14000, followed by stirring in ultrapure water. The entire amount of the dialysate (ultrapure water) was replaced three times between the beginning of the dialysis and 36 hours thereafter, to produce an HA/CMC condensation product-1 (crosslinked polymer material) (condensation product 1).

1-1-2. Production of HA/CMC Condensation Product-2

4400 mg of hyaluronic acid (HA) (available from Kewpie Corporation; molecular weight: 2200000) and 2000 mg of carboxymethyl cellulose (CMC) (available from Tokyo Chemical Industry Co., Ltd.; molecular weight: 1500000; degree of etherification: 0.5 to 0.8) were weighed. HA was added to a beaker containing 800 mL of ultrapure water and dissolved therein, and then CMC was added and dissolved therein, to prepare an HA/CMC solution. 8500 mg of ethyl-3-(3-dimethylaminopropyl)carbodiimide (EDC) (available from Dojindo Laboratories) was weighed and dissolved in 20.0 mL of ultrapure water, to prepare an EDC solution. While the HA/CMC solution was stirred at a stirring speed of 800 rpm at 80°C, 0.1 N HCl was added to the solution to adjust the pH to 4.50 to 5.20, and the entire amount of the EDC solution was added. After the addition, the resultant mixture was stirred for approximately 120 minutes until the pH remained unchanged, to produce a reaction solution. Finally, the reaction solution after the stirring was added to a dialysis membrane having a molecular weight cut off of 12000 to 14000, followed by stirring in ultrapure water. The dialysate (ultrapure water) was replaced three times between the beginning of the dialysis and 36 hours thereafter, to produce an HA/CMC condensation product-2 (condensation product 2).

1-1-3. Production of HA/CMC Condensation Product-3

An HA/CMC condensation product-3 (condensation product 3) was produced in the same manner as in the production of the HA/CMC condensation product-1 except that the EDC solution was added while the HA/CMC solution was stirred at a stirring speed of 200 rpm.

1-1-4. Production of HA/CMC Condensation Product-4

An HA/CMC condensation product-4 (condensation product 4) was produced in the same manner as in the production of the HA/CMC condensation product-1 except that the EDC solution was added while the HA/CMC solution was stirred at a stirring speed of 800 rpm.

1-1-5. Production of HA/CMC Condensation Product-5

An HA/CMC condensation product-5 (condensation product 5) was produced in the same manner as in the production of the HA/CMC condensation product-1 except that the EDC solution was added while the HA/CMC solution was stirred at 60°C.

1-1-6. Production of HA/CMC Condensation Product-6

An HA/CMC condensation product-6 (condensation product 6) was produced in the same manner as in the production of the HA/CMC condensation product-1 except that the EDC solution was added while the HA/CMC solution was stirred at 40°C.

The concentrations of the condensation product 1 to the condensation product 6 ((the masses of HA and CMC used as raw materials)/(the masses of HA, CMC, and water used during reaction)) were all 0.77 wt%.

1-2. Production of Medical Film

A biodegradable resin shown in Table 1 was provided. A powdery biodegradable resin that had been passed through a sieve having an opening of 38 $\mu$m was used. A nonwoven fabric-like biodegradable resin having a basis weight of 10.5 g/m$^2$ was used.

[Table 1]

| Resin composition | Name of biodegradable resin | Raw material composition (mass%) | | Number average molecular weight (Da) | Particle size ($\mu$m) |
|---|---|---|---|---|---|
| | | Glycolic acid | Lactic acid | | |
| Resin 1 | Polyglycolic acid (PGA) Powder | 100 | 0 | 5000 | 2.56 |
| Resin 2 | Polyglycolic acid (PGA) Powder | 100 | 0 | 11000 | 4.62 |
| Resin 3 | Polyglycolic acid (PGA) Powder | 100 | 0 | 164000 | 9.85 |
| Resin 4 | Polyglycolic acid (PGA) Nonwoven fabric | 100 | 0 | 53000 | Not measured |
| Resin 5 | Polyglycolic acid-polylactic acid copolymer (PGLA) Powder | 21 | 79 | 4000 to 15000 | 6.32 |
| Resin 6 | Polyglycolic acid-polylactic acid copolymer (PGLA) Powder | 45 | 55 | 7000 to 17000 | 7.48 |
| Resin 7 | Polylactic acid (PLA) powder | 0 | 100 | 100000 | 6.79 |
| Resin 8 | Polylactic acid (PLA) powder | 0 | 100 | 11000 | 5.31 |

1-2-1. Production of Film 1

100 g of the HA/CMC condensation product-1 was weighed and added to a beaker. 210.5 mg of the resin 3 was weighed and added to the HA/CMC condensation product-1 in the beaker and then stirred. A 11 × 11 cm frame was produced on a glass plate, and the condensation product 1 to which PGA powder had been added was poured into the frame. Drying was performed at a temperature of 30°C for approximately 3 days. After the drying, heat treatment was performed by using a hot pressing machine at 90°C for 15 minutes and then at 120°C for 10 minutes, to produce a film 1.

1-2-2. Production of Film 2

A film 2 was produced in the same manner as in the production of the film 1 except that the resin 3 was replaced with the resin 2.

1-2-3. Production of Film 3

100 g of the condensation product 1 was weighed and added to a beaker. The content of the beaker was stirred. A 11 × 11 cm frame was produced on a glass plate, and 50 g of the condensation product 1 was poured into the frame. The resin 4 in the form of nonwoven fabric was cut into three pieces each having a size of 11 × 11 cm, and the three pieces (approximately 210.0 mg) were stacked on the condensation product 1 in the frame without forming any gap, to thereby cover the condensation product 1. After confirmation that the PGA nonwoven fabric was impregnated with the HA/CMC condensation product-1, 50 g of the remaining HA/CMC condensation product-1 was poured into the frame. Drying was performed at a temperature of 30°C for approximately 3 days. After the drying, heat treatment was performed by using a hot pressing machine at 90°C for 15 minutes and then at 120°C for 10 minutes, to produce a film 3.

1-2-4. Production of Film 4

A film 4 was produced in the same manner as in the production of the film 1 except that the resin 3 was replaced with the resin 5.

1-2-5. Production of Film 5

A film 5 was produced in the same manner as in the production of the film 1 except that the resin 3 was replaced with the resin 6.

1-2-6. Production of Film 6

A film 6 was produced in the same manner as in the production of the film 1 except that the resin 3 was replaced with the resin 1.

1-2-7. Production of Film 7

A film 7 was produced in the same manner as in the production of the film 1 except that the condensation product 1 was replaced with the condensation product 3.

1-2-8. Production of Film 8

A film 8 was produced in the same manner as in the production of the film 1 except that the condensation product 1 was replaced with the condensation product 6.

1-2-9. Production of Film 9

A film 9 was produced in the same manner as in the production of the film 1 except that the amount of the resin 3 added was changed to 701.7 mg.

1-2-10. Production of Film 10

A film 10 was produced in the same manner as in the production of the film 1 except that the amount of the resin 3 added was changed to 70.2 mg.

1-2-11. Production of Film 11

A film 11 was produced in the same manner as in the production of the film 1 except that the amount of the resin 3 added was changed to 140.3 mg.

1-2-12. Production of Film 12

A film 12 was a commercially available medical film (Seprafilm, available from Baxter; "Seprafilm" is a registered trademark of Baxter International Inc.).

1-2-13. Production of Film 13

A film 13 was produced in the same manner as in the production of the film 1 except that no biodegradable resin was added.

1-2-14. Production of Film 14

A film 14 was produced in the same manner as in the production of the film 1 except that the resin 3 was replaced with the resin 7.

1-2-15. Production of Film 15

90 g of the condensation product 2 was weighed and added to a beaker. 252.0 mg of the resin 8 was weighed and added to the beaker, and the content in the beaker was stirred. A 11 × 11 cm frame was produced by using a silicone rubber on a glass plate, and the condensation product 2 to which PGA powder had been added was poured into the frame. After drying by air at room temperature, heat treatment was performed by using a hot pressing machine at 90°C for 15 minutes and then at 120°C for 10 minutes, to produce a film 15.

1-2-16. Production of Film 16

A film 16 was produced in the same manner as in the production of the film 15 except that no biodegradable resin was added.

1-2-17. Production of Film 17

A film 17 was produced in the same manner as in the production of the film 1 except that the condensation product 4 was used.

1-2-18. Production of Film 18

A film 18 was produced in the same manner as in the production of the film 1 except that the condensation product 5 was used.

Table 2 shows the type, concentration, and amount of the HA/CMC condensation product added, the type and amount of the biodegradable resin added, and the parts by weight of the biodegradable resin relative to 100 parts by mass of the HA/CMC used in production of samples.

[Table 2]

| | Sample | HA/CMC condensation product | | | Biodegradable resin | | Parts by weight of biodegradable resin relative to 100 parts by weight of HA/CMC |
| | | Type | Concentration (wt%) | Addition amount (g) | Type | Addition amount (mg) | |
|---|---|---|---|---|---|---|---|
| Example 1 | Film 1 | Condensation product 1 | 0.77 | 100 | Resin 3 | 210.5 | 27.3 |
| Example 2 | Film 2 | Condensation product 1 | 0.77 | 100 | Resin 2 | 210.5 | 27.3 |
| Example 3 | Film 3 | Condensation product 1 | 0.77 | 100 | Resin 4 | 210.0 | 27.3 |
| Example 4 | Film 4 | Condensation product 1 | 0.77 | 100 | Resin 5 | 210.5 | 27.3 |
| Example 5 | Film 5 | Condensation product 1 | 0.77 | 100 | Resin 6 | 210.5 | 27.3 |
| Example 6 | Film 6 | Condensation product 1 | 0.77 | 100 | Resin 1 | 210.5 | 27.3 |
| Example 7 | Film 7 | Condensation product 3 | 0.77 | 100 | Resin 3 | 210.5 | 27.3 |
| Example 8 | Film 8 | Condensation product 6 | 0.77 | 100 | Resin 3 | 210.5 | 27.3 |
| Example 9 | Film 9 | Condensation product 1 | 0.77 | 100 | Resin 3 | 701.7 | 91.1 |

(continued)

| | Sample | HA/CMC condensation product | | | Biodegradable resin | | Parts by weight of biodegradable resin relative to 100 parts by weight of HA/CMC |
|---|---|---|---|---|---|---|---|
| | | Type | Concentration (wt%) | Addition amount (g) | Type | Addition amount (mg) | |
| Example 10 | Film 10 | Condensation product 1 | 0.77 | 100 | Resin 3 | 70.2 | 9.1 |
| Example 11 | Film 11 | Condensation product 1 | 0.77 | 100 | Resin 3 | 140.3 | 18.2 |
| Comparative Example 1 | Film 12 | - | - | - | No | No | No |
| Comparative Example 2 | Film 13 | Condensation product 1 | 0.77 | 100 | No | No | No |
| Comparative Example 3 | Film 14 | Condensation product 1 | 0.77 | 100 | Resin 7 | 210.5 | 27.3 |
| Comparative Example 4 | Film 15 | Condensation product 2 | 0.77 | 90 | Resin 8 | 252.0 | 36.4 |
| Comparative Example 5 | Film 16 | Condensation product 2 | 0.77 | 90 | No | No | No |
| Comparative Example 6 | Film 17 | Condensation product 4 | 0.77 | 100 | Resin 3 | 210.5 | 27.3 |
| Comparative Example 7 | Film 18 | Condensation product 5 | 0.77 | 100 | Resin 3 | 210.5 | 27.3 |

2. Evaluation

2-1. Degree of Swelling

The film 1 to the film 18 were each cut into a size of 1 cm $\times$ 1 cm by using a rotary cutter to prepare a sample.

6.24 g of sodium dihydrogenphosphate dihydrate and 14.33 g of disodium hydrogenphosphate dodecahydrate were each dissolved in 0.2 L of ultrapure water to prepare a 0.2 M solution. Thereafter, the resultant solutions were mixed to prepare a PB solution having a pH of 6.0.

Each sample and a ruler were arranged together on a blue board, and they were photographed in such a manner that the contour of the sample was clear.

5 mL of the prepared PB solution was added to a 10 mL vial, and the sample was immersed in the PB solution for 10 seconds. Thereafter, the sample was taken out and arranged together with a ruler on a blue board, and the contour of the sample was photographed. The sample was returned to the PB solution after the photographing and immersed in the PB solution for 30 seconds. Thereafter, the sample was taken out and arranged together with a ruler on a blue board, and the contour of the sample was photographed. After 60 seconds of the immersion, the sample was taken out and arranged together with a ruler on a blue board, and the contour of the sample was photographed. After 300 seconds of the immersion, the sample was taken out and arranged together with a ruler on a blue board, and the contour of the sample was photographed. After 900 seconds of the immersion, the sample was taken out and arranged together with a ruler on a blue board, and the contour of the sample was photographed.

Based on the photographed image, the lengths of four sides of the sample were determined. The average value of the lengths of the four sides of the sample before the immersion in the PB solution was defined as $I_0$, and the average value of the lengths of the four sides after the elapse of each of the immersion times was defined as $I_1$. The ratio of these values ($I_1^3/I_0^3$) was calculated, and the ratio was defined as the degree of swelling after the elapse of each of the immersion times.

The degree of swelling of each sample is shown in Table 1.

[Table 3]

| | Sample | Degree of swelling | | | | |
|---|---|---|---|---|---|---|
| | | 10 seconds q1 (V1/V0) | 30 seconds q4 (V4/V0) | 60 seconds q2 (V2/V0) | 300 seconds q6 (V6/V0) | 900 seconds q3 (V3/V0) |
| Example 1 | Film 1 | 1.2 | 1.5 | 1.7 | 1.8 | 1.9 |
| Example 2 | Film 2 | 1.3 | 1.5 | 1.7 | 1.8 | 1.9 |
| Example 3 | Film 3 | 1.0 | 1.1 | 1.4 | 1.6 | 2.0 |
| Example 4 | Film 4 | 1.1 | 1.5 | 1.8 | 2.3 | 2.4 |
| Example 5 | Film 5 | 1.1 | 1.5 | 1.7 | 2.1 | 2.2 |
| Example 6 | Film 6 | 1.3 | 1.6 | 1.9 | 1.9 | 1.8 |
| Example 7 | Film 7 | 1.4 | 1.6 | 1.9 | 1.9 | 1.8 |
| Example 8 | Film 8 | 1.1 | 1.5 | 1.8 | 2.1 | 2.1 |
| Example 9 | Film 9 | 1.1 | 1.7 | 1.9 | 2.4 | 2.4 |
| Example 10 | Film 10 | 1.1 | 1.5 | 1.7 | 2.2 | 2.3 |
| Example 11 | Film 11 | 1.1 | 1.5 | 1.7 | 2.4 | 2.4 |
| Comparative Example 1 | Film 12 | 1.7 | 1.8 | 1.9 | 1.9 | 2.0 |
| Comparative Example 2 | Film 13 | 1.6 | 1.7 | 1.9 | 2.0 | 2.1 |
| Comparative Example 3 | Film 14 | 1.6 | 2.1 | 2.4 | 2.6 | 2.6 |
| Comparative Example 4 | Film 15 | 1.6 | 1.7 | 1.9 | 2.2 | 2.2 |
| Comparative Example 5 | Film 16 | 1.7 | 2.0 | 2.2 | 2.4 | 2.6 |
| Comparative Example 6 | Film 17 | 1.7 | 1.8 | 2.0 | 2.3 | 2.3 |
| Comparative Example 7 | Film 18 | 1.6 | 2.0 | 2.4 | 2.4 | 2.6 |

Discussion

The results in Table 3 indicate that each of the films 1 to 11 has suppressed initial degree of swelling, but exhibits a high degree of swelling after the elapse of a predetermined period of time.

2-2-1. Ease in Re-attachment

A simulated organ (VTT-STANDARD-TYPE, VTT-STD, available from Kotobuki Medical Inc.) was adequately moistened with ultrapure water, and water on the surface was wiped using Kimtowel. Thereafter, each of the films 1 to 18 having a size of 1 × 2 cm was disposed on the simulated organ and allowed to stand for 30 seconds, and then the film was released from the simulated organ and re-attached to an adjacent portion on the same simulated organ. Ease in re-attachment in this case was evaluated by five monitors on a scale of 1 to 9, wherein 1 indicated difficult re-attachment and 9 indicated easy re-attachment. The average value of the evaluation results by the monitors was used as an index of ease in re-attachment of each of the films.

2-2-2. Ease in Spreading After Releasing

A simulated organ (VTT-STANDARD-TYPE, VTT-STD, available from Kotobuki Medical Inc.) was adequately moistened with ultrapure water, and water on the surface was wiped using Kimtowel. Thereafter, 200 μL of ultrapure water was applied onto the entire surface of the simulated organ. Each of the films 1 to 18 having a size of 1 × 3 cm was disposed on the simulated organ and allowed to stand for 30 seconds, and then the film was released from the simulated organ and re-attached to an adjacent portion on the same simulated organ. The condition of the film when the film was re-attached was evaluated by five monitors. The average value of the evaluation results by the monitors was used as an index of ease in spreading after releasing of each of the films. The evaluation criteria were as follows. A score of 4 or higher was defined as being easy to re-attach.

1: Spreading was impossible or breakage occurred.
2: Spreading required a time of 90 seconds or longer.
3: Spreading was performed within 30 to 90 seconds.
4: The entire film was curled but spread within 30 seconds.
5: Spreading was performed although some of the film was curled.

The evaluation results of ease in re-attachment and ease in spreading after releasing are shown in Table 4.

[Table 4]

|  | Sample | Ease in re-attachment | Ease in spreading after releasing |
|---|---|---|---|
| Example 1 | Film 1 | 9 | 5 |
| Example 2 | Film 2 | 9 | 5 |
| Example 3 | Film 3 | 9 | 5 |
| Example 4 | Film 4 | 9 | 4 |
| Example 5 | Film 5 | 9 | 5 |
| Example 6 | Film 6 | 9 | 4 |
| Example 7 | Film 7 | 8 | 4 |
| Example 8 | Film 8 | 9 | 5 |
| Example 9 | Film 9 | 9 | 4 |
| Example 10 | Film 10 | 9 | 5 |
| Example 11 | Film 11 | 9 | 5 |
| Comparative Example 1 | Film 12 | 1 | 1 |
| Comparative Example 2 | Film 13 | 3 | 2 |
| Comparative Example 3 | Film 14 | 4 | 2 |
| Comparative Example 4 | Film 15 | 4 | 3 |
| Comparative Example 5 | Film 16 | 1 | 1 |
| Comparative Example 6 | Film 17 | 4 | 3 |
| Comparative Example 7 | Film 18 | 4 | 2 |

Discussion

The results in Table 4 indicate that each of the films 1 to 11 has a low initial degree of swelling, has good re-attachability, and is readily spread after being attached once and released. According to the knowledge of the present inventors, it is conceived that the degree of swelling of the film when water is applied on one surface thereof and allowed to stand for 30 seconds corresponds to the degree of swelling of the film when its entirety is immersed in the PB solution for 10 seconds. Thus, the results in Table 3 and Table 4 indicate that the medical film having a low degree of swelling when immersed in the PB solution for 10 seconds has high re-attachability.

2-3. Tensile Strength

The film 1, films 5 and 6, and films 9 to 13 (film 12 was Seprafilm (available from Baxter)) were each cut into a strip form having a long side of 40 mm and a short side of 10 mm to prepare a sample. Using a creepmeter (RE2-33005B, available from Yamaden Co., Ltd.), the tensile strength of the sample wet with ultrapure water was measured. The sample was fixed to chucks in such a manner that the distance between chucks became 20 mm, and Bell Clean (product number: E-2, available from AION Co., Ltd.) containing ultrapure water was attached to a 10 mm $\times$ 10 mm center portion of one surface of the sample and allowed to stand for 10 seconds. After the elapse of 10 seconds, the sample was pulled at 0.1 mm/sec, to measure the tensile strength (MPa).
The results of measurement of the tensile strength of the samples are shown in Table 5.

[Table 5]

|  | Sample | Tensile strength (MPa) |
| --- | --- | --- |
| Example 1 | Film 1 | 7.07 |
| Example 5 | Film 5 | 33.30 |
| Example 6 | Film 6 | 18.10 |
| Example 9 | Film 9 | 5.86 |
| Example 10 | Film 10 | 6.71 |
| Example 11 | Film 11 | 13.01 |
| Comparative Example 1 | Film 12 | 0.66 |
| Comparative Example 2 | Film 13 | 1.54 |

Discussion

The results in Table 5 indicate that the medical film having a low initial degree of swelling is less likely to undergo a reduction in strength even when the medical film is attached to an organ, and is easily released from and re-attached to the organ. The crosslinked polymer material, which is a component of the medical film, swells when it absorbs water and turns into a gel. This is probably because the medical film undergoes a gradual reduction in strength due to a low initial degree of swelling, although the strength decreases when the film turns into a gel. Furthermore, it is conceived that the films 5 and 6, which contained the low-molecular-weight resin 6 and resin 1, respectively, exhibited a high tensile strength because the amount of sustained release of the acid was increased, and the medical film underwent a gradual reduction in strength.

2-4. Adhesive Strength

The film 1, films 5 and 6, film 12 (Seprafilm (available from Baxter)), and film 13 were each cut into a strip form having a long side of 50 mm and a short side of 10 mm to prepare a sample. Using a creepmeter (RE2-33005B, available from Yamaden Co., Ltd.), the adhesive strength to a surface of a simulated organ (silicone rubber sheet (thickness: 3 mm), product number: 6-611-05, available from As One Corporation) wet with ultrapure water was measured. In this case, a mending tape (product number: 810-1-18, available from 3M Japan Limited; base material: acetate film; adhesive agent: acrylic) was attached onto the surface of each sample opposite to the surface to be brought into contact with the simulated organ. 5 mm of a short side of the measurement sample was fixed to one pulling chuck, and the simulated organ was fixed to the other pulling chuck. The sample having a long side of 40 mm and a short side of 10 mm was attached to the wet simulated organ and allowed to stand for 900 seconds. After the elapse of 900 seconds, the sample was pulled at 0.05 mm/sec to measure the adhesive strength (N). For Comparative Examples, the measurement was performed on INTERCEED (available from Johnson & Johnson) and TENALEAF (available from Gunze Limited), which are commercially available synthetic adhesion barriers.

The results of measurement of the adhesive strength of the samples are shown in Table 6.

[Table 6]

|  | Sample | Adhesive strength (N) |
| --- | --- | --- |
| Example 1 | Film 1 | 2.91 |
| Example 5 | Film 5 | 3.65 |
| Example 6 | Film 6 | 5.07 |
| Comparative Example 1 | Film 12 | 2.95 |
| Comparative Example 2 | Film 13 | 2.37 |
| Reference 1 | INTERCEED | 0.03 |
| Reference 2 | TENALEAF | 1.52 |

Discussion

The results in Table 6 indicate that the film 1, film 5, and film 6 exhibited high adhesive strength, but a difference in adhesive strength was observed between the films. This is probably because the glycolic acid component contained in the glycolic acid (co)polymer releases an acid when brought into contact with water, and the acid affects suppression of the decomposition of the crosslinked polymer material. In the film 6, the molecular weight of the glycolic acid (co)polymer is lower than that in the film 1, and the glycolic acid content in the glycolic acid (co)polymer is higher than that in the film 5, and thus the amount of the released acid became larger, resulting in suppression of the decomposition of the crosslinked polymer material. Therefore, conceivably, the film 6 exhibits a higher adhesive strength than the film 1 exhibiting a similar degree of swelling after the elapse of 900 seconds, and exhibits high adhesive strength even though the degree of swelling is lower than that of the film 5.

Experiment 2

In Experiment 2, the relationship between the thickness and degree of swelling of the medical film was examined by using the film 1, the film 6, and films having thicknesses different from those of the films 1 and 6.

1. Production of Medical Film

1-1. Film 1-2

53 g of the condensation product 1 was weighed and added to a beaker. 111.6 mg of the resin 3 was weighed and added to the beaker and then stirred. A 11 × 11 cm frame was produced on a glass plate, and the condensation product 1 to which the resin 3 had been added was poured into the frame. Drying was performed at a temperature of 30°C for approximately 3 days. After the drying, heat treatment was performed by using a hot pressing machine at 90°C for 15 minutes and then at 120°C for 10 minutes, to produce a film 1-2.

1-2. Film 1-3

90 g of the condensation product 1 was weighed and added to a beaker. 189.5 mg of the resin 3 was weighed and added to the beaker, and a film 1-3 was produced in the same manner as the film 1-2.

1-3. Film 1-4

200 g of the condensation product 1 was weighed and added to a beaker. 421 mg of the resin 3 was weighed and added to the beaker, and a film 1-3 was produced in the same manner as the film 1-2.

1-4. Film 6-2

50 g of the condensation product 1 was weighed and added to a beaker. Then, a film 6-2 was produced in the same manner as in the production of the film 1-2 except that the resin 3 was not added.

1-5. Film 6-3

75 g of the condensation product 1 was weighed and added to a beaker. Then, a film 6-3 was produced in the same manner as in the production of the film 1-2 except that the resin 3 was not added.

2. Measurement

The degree of swelling of each of the film 1, film 1-2 to film 1-4, film 13, film 13-2, and film 13-3 was measured in the same manner as in Experiment 1.

The results of measurement of the degrees of swelling of the samples are shown in Table 7.

[Table 7]

| Sample | Degree of swelling | | | | | Note |
|---|---|---|---|---|---|---|
| | 10 seconds q1 (V1/V0) | 30 seconds q4 (V4/V0) | 60 seconds q2 (V2/V0) | 300 seconds q6 (V6/V0) | 900 seconds q3 (V3/V0) | |
| Film 1 | 1.2 | 1.5 | 1.7 | 1.8 | 1.9 | Thickness 80 μm |
| Film 1-2 | 1.4 | 1.8 | 1.9 | 1.9 | 1.9 | Thickness 60 μm |

(continued)

| Sample | Degree of swelling | | | | | Note |
|---|---|---|---|---|---|---|
| | 10 seconds q1 (V1/V0) | 30 seconds q4 (V4/V0) | 60 seconds q2 (V2/V0) | 300 seconds q6 (V6/V0) | 900 seconds q3 (V3/V0) | |
| Film 1-3 | 1.2 | 1.5 | 1.7 | 1.9 | 1.9 | Thickness 75 $\mu$m |
| Film 1-4 | 1.0 | 1.2 | 1.4 | 1.9 | 2.3 | Thickness 215 $\mu$m |
| Film 6 | 1.6 | 1.7 | 1.9 | 2.0 | 2.1 | Thickness 55 $\mu$m |
| Film 6-2 | 2.7 | 2.8 | 2.7 | 2.7 | 2.7 | Thickness 28 $\mu$m |
| Film 6-3 | 2.0 | 2.4 | 2.4 | 2.4 | 2.4 | Thickness 42 $\mu$m |

As shown in Table 7, a larger thickness resulted in a lower initial degree of swelling of the medical film.

The present application claims priority to Japanese Patent Application No. 2023-077267 filed on May 9, 2023. The original specification of the application, and the matters described in the claims are incorporated herein by reference.

**INDUSTRIAL APPLICABILITY**

The medical film according to the present invention has good re-attachability to an affected part.

**Claims**

1. A medical film comprising a crosslinked polymer material and a biodegradable resin, wherein
   the medical film has a ratio q1 (V1/V0) of a volume V1 after 10 seconds of immersion in a phosphate buffer (PB) having a pH of 6.0 and a concentration of 0.2 M to a volume V0 before the immersion of 1.5 or less.

2. The medical film according to claim 1, wherein
   the medical film has a ratio q2 (V2/V0) of a volume V2 after 60 seconds of immersion in a phosphate buffer (PB) having a pH of 6.0 and a concentration of 0.2 M to a volume V0 before the immersion of 1.9 or less.

3. The medical film according to claim 1 or 2, wherein
   the medical film has a ratio q3 (V3/V0) of a volume V3 after 900 seconds of immersion in a phosphate buffer (PB) having a pH of 6.0 and a concentration of 0.2 M to a volume V0 before the immersion of 1.8 or more.

4. The medical film according to any one of claims 1 to 3, wherein
   the medical film contains 5 parts by mass or more and 95 parts by mass or less of the biodegradable resin relative to 100 parts by mass of the crosslinked polymer material.

5. The medical film according to any one of claims 1 to 4, wherein
   the biodegradable resin is a polymer in which a proportion of a constituent unit derived from glycolic acid is 5 mass% or more and 100 mass% or less relative to a mass of the entire biodegradable resin.

6. The medical film according to any one of claims 1 to 5, wherein
   the medical film has a thickness of 15 $\mu$m or more and 500 $\mu$m or less.

7. The medical film according to any one of claims 1 to 6, wherein
   the medical film is an adhesion barrier.

## INTERNATIONAL SEARCH REPORT

| International application No. |
|---|
| **PCT/JP2024/017249** |

### A. CLASSIFICATION OF SUBJECT MATTER

*A61L 15/28*(2006.01)i; *A61L 15/26*(2006.01)i; *A61L 15/64*(2006.01)i
FI:   A61L15/28 100; A61L15/26 100; A61L15/64 100

According to International Patent Classification (IPC) or to both national classification and IPC

### B. FIELDS SEARCHED

Minimum documentation searched (classification system followed by classification symbols)

A61L15/00-15/64; A61K9/00-9/72; A61K47/00-47/69; C08K3/00-13/08; C08L1/00-101/14; C08L101/16; C08J5/00-5/02; C08J5/12-5/22

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
|---|---|---|
| Y | WO 2021/201150 A1 (KUREHA CORPORATION) 07 October 2021 (2021-10-07) claims 1, 3, 6-9, paragraphs [0011], [0022], [0027], [0030], [0031], [0042], [0057], [0059], [0065], [0073], [0085], [0088] | 1-7 |
| Y | JP 2020-139117 A (DAI NIPPON PRINTING CO., LTD.) 03 September 2020 (2020-09-03) claims 1, 9, paragraphs [0129], [0130], [0176], [0177] | 1-7 |

☐ Further documents are listed in the continuation of Box C.   ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **21 June 2024** | **16 July 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

**INTERNATIONAL SEARCH REPORT**
Information on patent family members

International application No.

**PCT/JP2024/017249**

| Patent document cited in search report | | | Publication date (day/month/year) | Patent family member(s) | Publication date (day/month/year) |
|---|---|---|---|---|---|
| WO | 2021/201150 | A1 | 07 October 2021 | US 2023/0119326 A1 claims 1, 3, 6-9, paragraphs [0042], [0044], [0047], [0048], [0059], [0074], [0076], [0082], [0098], [0110], [0113], [0116] | |
| JP | 2020-139117 | A | 03 September 2020 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011081162 A
- WO 2017164264 A
- JP 2023077267 A